# EUROPEAN PATENT SPECIFICATION

(11) **EP 1 693 068 B1**
(45) Date of publication and mention of the grant of the patent: **04.05.2016**
(21) Application number: 05077510.5
(22) Date of filing: 16.11.2001
(51) Int. Cl.: A61K 39/39, A61K 39/04, A61P 19/02

(54) **Use of M. tuberculosis chaperonin 60.1 for the treatment of arthritis**
Verwendung von Chaperonin 60.1 zur Behandlung von Arthritis
Utilisation de la chaperonine 60.1 du M. Tuberculosis pour le traitement de l'arthrite

(30) Priority: 17.11.2000 GB 0028122
(43) Date of publication of application: 23.08.2006
(62) Divisional of application: 01983694.9
(73) Proprietor: Peptinnovate Limited, London WC1V 6XX (GB)
(72) Inventor: Coates, Anthony Robert Milnes Helperby Therap. Ltd, Cranmer Terrace London, SW17 0RE (GB)
(74) Representative: D Young & Co LLP

(56) References cited:
- WO-A-00/27870
- WO-A-95/25744
- KONG T H ET AL: "Mycobacterium tuberculosis expresses two chaperonin-60 homologs" PROCEEDINGS OF THE NATIONAL ACADEMY OF SCIENCES OF USA, NATIONAL ACADEMY OF SCIENCE, WASHINGTON, DC, US, vol. 90, April 1993 (1993-04), pages 2608-2612, XP002140326 ISSN: 0027-8424
- QUAYLE A J ET AL: "PEPTIDE RECOGNITION, T CELL RECEPTOR USAGE AND HLA RESTRICTION ELEMENTS OF HUMAN HEAT-SHOCK PROTEIN (HSP) 60 AND MYCOBACTERIAL 65-KDA HSP-REACTIVE T CELL CLONES FROM RHEUMATOID SYNOVIAL FLUID" EUROPEAN JOURNAL OF IMMUNOLOGY, WEINHEIM, DE, vol. 22, May 1992 (1992-05), pages 1315-1322, XP000989540 ISSN: 0014-2980
- ANDERTON S M ET AL: "ACTIVATION OF T CELLS RECOGNIZING SELF 60-KD HEAT SHOCK PROTEIN CAN PROTECT AGAINST EXPERIMENTAL ARTHRITIS" JOURNAL OF EXPERIMENTAL MEDICINE, TOKYO, JP, vol. 181, no. 3, 1 March 1995 (1995-03-01), pages 943-952, XP000881729 ISSN: 0022-1007
- COBELENS PIETER M ET AL: "Treatment of adjuvant-induced arthritis by oral administration of mycobacterial Hsp65 during disease" ARTHRITIS AND RHEUMATISM, vol. 43, no. 12, December 2000 (2000-12), pages 2694-2702, XP002387789 ISSN: 0004-3591
- PRAKKEN B J ET AL: "PEPTIDE-INDUCED NASAL TOLERANCE FOR A MYCOBACTERIAL HEAT SHOCK PROTEIN 60 T CELL EPITOPE IN RATS SUPPRESSES BOTH ADJUVANT ARTHRITIS AND NONMICROBIALLY INDUCED EXPERIMENTAL ARTHRITIS" PROCEEDINGS OF THE NATIONAL ACADEMY OF SCIENCES OF USA, NATIONAL ACADEMY OF SCIENCE, WASHINGTON, DC, US, vol. 94, 1 April 1997 (1997-04-01), pages 3284-3289, XP000882776 ISSN: 0027-8424
- PRAKKEN B ET AL: "NASAL ADMINISTRATION OF ARTHRITIS-RELATED T CELL EPITOPES OF HEAT SHOCK PROTEIN 60 AS A PROMISING WAY FOR IMMUNOTHERAPY IN CHRONIC ARTHRITIS" BIOTHERAPY, KLUWER ACADEMIC PUBLISHERS, DORDRECHT, NL, vol. 10, no. 3, 1998, pages 205-211, XP000881736 ISSN: 0921-299X
- LEWTHWAITE JO C ET AL: "Mycobacterium tuberculosis chaperonin 60.1 is a more potent cytokine stimulator than chaperonin 60.2 (Hsp 65) and contains a CD14-binding domain" INFECTION AND IMMUNITY, vol. 69, no. 12, December 2001 (2001-12), pages 7349-7355, XP002387790 ISSN: 0019-9567
- VANDOOREN BERNARD ET AL: "The abundant synovial expression of the RANK/RANKL/osteoprotegerin system in peripheral spondylarthritis is partially disconnected from inflammation", ARTHRITIS & RHEUMATISM, vol. 58, no. 3, March 2008 (2008-03), pages 718-729, ISSN: 0004-3591
- CONWAY JAMES G ET AL: "Effects of the cFMS kinase inhibitor 5-(3-methoxy-4-((4-methoxybenzyl )oxy)benzyl)pyrimidine-2,4-diamine (GW2580) in normal and arthritic rats.", THE JOURNAL OF PHARMACOLOGY AND EXPERIMENTAL THERAPEUTICS JUL 2008 LNKD- PUBMED:18434589, vol. 326, no. 1, July 2008 (2008-07), pages 41-50, ISSN: 1521-0103
- BAIRD P N ET AL: "CLONING AND SEQUENCE ANALYSIS OF THE 10 KDA ANTIGEN GENE OF MYCOBACTERIUM TUBERCULOSIS", JOURNAL OF GENERAL MICROBIOLOGY, SOCIETY FOR MICROBIOLOGY, READING, GB, vol. 135, no. 4, 1 January 1989 (1989-01-01), pages 931-939, XP008005161, ISSN: 0022-1287

### SEQUENCE LISTING

<110> Helperby Therapeutics Limited
<120> Biological materials and uses thereof
<130> HELBH/P25182EPdiv1
<140> EP05077510.5
   <141> 16 November 2001
<150> GB0028122.0
   <151> 17 November 2000
<160> 30
<170> SeqWin99
<210> 1
   <211> 8
   <212> PRT
   <213> Unknown
<220>
   <223> Polypeptide derived from various Mycobacterium species
<400> 1
   Met Ser Lys Leu Ile Glu Tyr Asp
   1               5
<210> 2
   <211> 8
   <212> PRT
   <213> Unknown
<220>
   <223> Polypeptide derived from various Mycobacterium species
<400> 2
   Ala Met Glu Val Gly Met Asp Lys
   1               5
<210> 3
   <211> 9
   <212> PRT
   <213> Unknown
<220>
   <223> Polypeptide derived from various Mycobacterium species
<400> 3
   Ala Lys Ala Phe Gly Gly Pro Thr Val
   1               5
<210> 4
   <211> 8
   <212> PRT
   <213> Unknown
<220>
   <223> Polypeptide derived from various Mycobacterium species
<400> 4
   Pro Phe Glu Asp Leu Gly Ala Gln
   1               5
<210> 5
   <211> 10
   <212> PRT
   <213> Unknown
<220>
   <223> Polypeptide derived from various Mycobacterium species
<400> 5
   Gln Ala Leu Ile Lys Gly Gly Leu Arg Leu
   1               5                   10
<210> 6
   <211> 20
   <212> PRT
   <213> Unknown
<220>
   <223> Polypeptide derived from various Mycobacterium species
<400> 6
   Val Asn Pro Ile Ala Leu Gly Val Gly Ile Gly Lys Ala Ala Asp Ala
   1               5                   10                  15
   Val Ser Glu Ala
               20
<210> 7
   <211> 12
   <212> PRT
   <213> Unknown
<220>
   <223> Polypeptide derived from various Mycobacterium species
<400> 7
   Ala Ser Ala Thr Pro Val Ser Gly Lys Thr Gly Ile
   1               5                   10
<210> 8
   <211> 12
   <212> PRT
   <213> Unknown
<220>
   <223> Polypeptide derived from various Mycobacterium species
<400> 8
   Ala Gln Val Ala Thr Val Ser Ser Arg Asp Glu Gln
   1               5                   10
<210> 9
   <211> 16
   <212> PRT
   <213> Unknown
<220>
   <223> Polypeptide derived from various Mycobacterium species
<400> 9
   Val Gly Glu Ala Met Ser Lys Val Gly His Asp Gly Val Val Ser Val
   1               5                   10                  15
<210> 10
   <211> 22
   <212> PRT
   <213> Unknown
<220>
   <223> Polypeptide derived from various Mycobacterium species
<400> 10
   Ser Thr Leu Gly Thr Glu Leu Glu Phe Thr Glu Gly Ile Gly Phe Asp
   1               5                   10                  15
   Lys Gly Phe Leu Ser Ala
               20
<210> 11
   <211> 25
   <212> PRT
   <213> Unknown
<220>
   <223> Polypeptide derived from various Mycobacterium species
<400> 11
   Lys Gly Phe Leu Ser Ala Tyr Phe Val Thr Asp Phe Asp Asn Gln Gln
   1               5                   10                  15
   Ala Val Leu Glu Asp Ala Leu Ile Leu
               20                  25
<210> 12
   <211> 14
   <212> PRT
   <213> Unknown
<220>
   <223> Polypeptide derived from various Mycobacterium species
<400> 12
   Phe Asp Asn Gln Gln Ala Val Leu Glu Asp Ala Leu Ile Leu
   1               5                   10
<210> 13
   <211> 9
   <212> PRT
   <213> Unknown
<220>
   <223> Polypeptide derived from various Mycobacterium species
<400> 13
   His Gln Asp Lys Ile Ser Ser Leu Pro
   1               5
<210> 14
   <211> 8
   <212> PRT
   <213> Unknown
<220>
   <223> Polypeptide derived from various Mycobacterium species
<400> 14
   Ala Ile Arg Lys Thr Leu Lys Ala
   1               5
<210> 15
   <211> 18
   <212> PRT
   <213> Unknown
<220>
   <223> Polypeptide derived from various Mycobacterium species
<400> 15
   Gly Pro Tyr Phe Gly Asp Arg Arg Lys Ala Phe Leu Glu Asp Leu Ala
   1               5                   10                  15
   Val Val
<210> 16
   <211> 16
   <212> PRT
   <213> Unknown
<220>
   <223> Polypeptide derived from various Mycobacterium species
<400> 16
   Val Asn Pro Asp Ala Gly Met Val Leu Arg Glu Val Gly Leu Glu Val
   1               5                   10                  15
<210> 17
   <211> 12
   <212> PRT
   <213> Unknown
<220>
   <223> Polypeptide derived from various Mycobacterium species
<400> 17
   Leu Gly Ser Ala Arg Arg Val Val Val Ser Lys Asp
   1               5                   10
<210> 18
   <211> 16
   <212> PRT
   <213> Unknown
<220>
   <223> Polypeptide derived from various Mycobacterium species
<400> 18
   Asp Thr Val Ile Val Asp Gly Gly Gly Thr Ala Glu Ala Val Ala Asn
   1               5                   10                  15
<210> 19
   <211> 11
   <212> PRT
   <213> Unknown
<220>
   <223> Polypeptide derived from various Mycobacterium species
<400> 19
   Arg Ala Lys His Leu Arg Ala Glu Ile Asp Lys
   1               5                   10
<210> 20
   <211> 13
   <212> PRT
   <213> Unknown
<220>
   <223> Polypeptide derived from various Mycobacterium species
<400> 20
   Val Gly Ala Ala Thr Glu Thr Ala Leu Lys Glu Arg Lys
   1               5                   10
<210> 21
   <211> 9
   <212> PRT
   <213> Unknown
<220>
   <223> Polypeptide derived from various Mycobacterium species
<400> 21
   Glu Ser Val Glu Asp Ala Val Ala Ala
   1               5
<210> 22
   <211> 23
   <212> PRT
   <213> Unknown
<220>
   <223> Polypeptide derived from various Mycobacterium species
<400> 22
   Pro Gly Gly Gly Ala Ser Leu Ile His Gln Ala Arg Lys Ala Leu Thr
   1               5                   10                  15
   Glu Leu Arg Ala Ser Leu Thr
               20
<210> 23
   <211> 16
   <212> PRT
   <213> Unknown
<220>
   <223> Polypeptide derived from various Mycobacterium species
<400> 23
   Gly Pro Glu Val Leu Gly Val Asp Val Phe Ser Glu Ala Leu Ala Ala
   1               5                   10                  15
<210> 24
   <211> 14
   <212> PRT
   <213> Unknown
<220>
   <223> Polypeptide derived from various Mycobacterium species
<400> 24
   Pro Leu Phe Trp Ile Ala Ala Asn Ala Gly Leu Asp Gly Ser
   1               5                   10
<210> 25
   <211> 8
   <212> PRT
   <213> Unknown
<220>
   <223> Polypeptide derived from various Mycobacterium species
<400> 25
   Val Val Val Asn Lys Val Ser Glu
   1               5
<210> 26
   <211> 15
   <212> PRT
   <213> Unknown
<220>
   <223> Polypeptide derived from various Mycobacterium species
<400> 26
   Val Asn Thr Leu Ser Tyr Gly Asp Leu Ala Ala Asp Gly Val Ile
   1               5                   10                  15
<210> 27
   <211> 26
   <212> PRT
   <213> Unknown
<220>
   <223> Polypeptide derived from various Mycobacterium species
<400> 27
   Arg Ser Ala Val Leu Asn Ala Ser Ser Val Ala Arg Met Val Leu Thr
   1               5                   10                  15
   Thr Glu Thr Val Val Val Asp Lys Pro Ala
               20                  25
<210> 28
   <211> 13
   <212> PRT
   <213> Unknown
<220>
   <223> Polypeptide derived from various Mycobacterium species
<400> 28
   Lys Ala Glu Asp His Asp His His His Gly His Ala His
   1               5                   10
<210> 29
   <211> 1620
   <212> DNA
   <213> Mycobacterium tuberculosis
<400> 29
   atgagcaagc tgatcgaata cgacgaaacc gcgcgtcgcg ccatggaggt cggcatggac    60
   aagctggccg acaccgtgcg ggtgacgctg gggccgcgcg gccggcatgt ggtgctggcc   120
   aaggcgtttg gcggacccac ggttaccaac gacggcgtca cggtggcacg tgagatcgag   180
   ctggaagatc cgtttgaaga cttgggcgcc cagctggtga agtcggtggc caccaagacc   240
   aacgatgtgg ccggtgacgg caccaccacc gcaaccatct tggcgcaggc actgatcaag   300
   ggcggcctga ggctagtggc cgccggcgtc aacccgatcg cgctcggcgt gggaatcggc   360
   aaggccgccg acgcggtatc cgaggcgctg ctggcatcgg ccacgccggt gtccggcaag   420
   accggcatcg cgcaggtggc gacggtgtcc tcgcgcgacg agcagatcgg tgacctggtt   480
   ggcgaagcga tgagcaaggt cggccacgac ggcgtggtca gcgtcgaaga atcctcgacg   540
   ctgggcaccg agttggagtt caccgagggt atcggcttcg acaagggctt cttgtcggca   600
   tacttcgtta ccgacttcga taaccagcag gcggtgctcg aggacgcgtt gatcctgctg   660
   caccaagaca agatcagctc gcttcccgat ctgttgccat tgctggaaaa ggttgcagga   720
   acgggtaagc cactactgat cgtggctgaa gacgtggagg gcgaagcgtt ggcgacgctg   780
   gtcgtcaacg cgattcgcaa gacgttgaaa gcggtcgcgg tcaaggggcc gtacttcggt   840
   gaccgccgta aggcgttcct tgaggacctg gcggtggtga cgggtggcca ggtggtcaac   900
   cccgacgccg gcatggtgct gcgcgaggtg ggcttggagg tgctgggctc ggcccgacgc   960
   gtggtggtca gcaaggacga cacggtcatt gtcgacggcg gcggcaccgc agaagcggtg  1020
   gccaaccggg cgaagcactt gcgtgccgag atcgacaaga gcgattcgga ttgggatcgg  1080
   gaaaagcttg gcgagcggct ggccaaactg gccggcgggg ttgctgtcat caaggtgggt  1140
   gccgccaccg agaccgcact caaggagcgc aaggaaagcg tcgaggatgc ggtcgcggcc  1200
   gccaaggccg cggtcgagga gggcatcgtc cctggtgggg gagcctcgct catccaccag  1260
   gcccgcaagg cgctgaccga actgcgtgcg tcgctgaccg gtgacgaggt cctcggtgtc  1320
   gacgtgttct ccgaagccct tgccgcgccg ttgttctgga tcgccgccaa cgctggcttg  1380
   gacggctcgg tggtggtcaa caaggtcagc gagctacccg ccgggcatgg gctgaacgtg  1440
   aacaccctga gctatggtga cttggccgct gacggcgtca tcgacccggt caaggtgact  1500
   aggtcggcgg tgttgaacgc gtcatcggtt gcccggatgg tactcaccac cgagacggtc  1560
   gtggtcgaca agccggccaa ggcagaagat cacgaccatc accacgggca cgcgcactga  1620
<210> 30
   <211> 539
   <212> PRT
   <213> Mycobacterium tuberculosis
<400> 30
   Met Ser Lys Leu Ile Glu Tyr Asp Glu Thr Ala Arg Arg Ala Met Glu
   1               5                   10                  15
   Val Gly Met Asp Lys Leu Ala Asp Thr Val Arg Val Thr Leu Gly Pro
               20                  25                  30
   Arg Gly Arg His Val Val Leu Ala Lys Ala Phe Gly Gly Pro Thr Val
           35                  40                  45
   Thr Asn Asp Gly Val Thr Val Ala Arg Glu Ile Glu Leu Glu Asp Pro
       50                  55                  60
   Phe Glu Asp Leu Gly Ala Gln Leu Val Lys Ser Val Ala Thr Lys Thr
   65                  70                  75                  80
   Asn Asp Val Ala Gly Asp Gly Thr Thr Thr Ala Thr Ile Leu Ala Gln
                   85                  90                  95
   Ala Leu Ile Lys Gly Gly Leu Arg Leu Val Ala Ala Gly Val Asn Pro
               100                 105                 110
   Ile Ala Leu Gly Val Gly Ile Gly Lys Ala Ala Asp Ala Val Ser Glu
           115                 120                 125
   Ala Leu Leu Ala Ser Ala Thr Pro Val Ser Gly Lys Thr Gly Ile Ala
       130                 135                 140
   Gln Val Ala Thr Val Ser Ser Arg Asp Glu Gln Ile Gly Asp Leu Val
   145                 150                 155                 160
   Gly Glu Ala Met Ser Lys Val Gly His Asp Gly Val Val Ser Val Glu
                   165                 170                 175
   Glu Ser Ser Thr Leu Gly Thr Glu Leu Glu Phe Thr Glu Gly Ile Gly
               180                 185                 190
   Phe Asp Lys Gly Phe Leu Ser Ala Tyr Phe Val Thr Asp Phe Asp Asn
           195                 200                 205
   Gln Gln Ala Val Leu Glu Asp Ala Leu Ile Leu Leu His Gln Asp Lys
       210                 215                 220
   Ile Ser Ser Leu Pro Asp Leu Leu Pro Leu Leu Glu Lys Val Ala Gly
   225                 230                 235                 240
   Thr Gly Lys Pro Leu Leu Ile Val Ala Glu Asp Val Glu Gly Glu Ala
                   245                 250                 255
   Leu Ala Thr Leu Val Val Asn Ala Ile Arg Lys Thr Leu Lys Ala Val
               260                 265                 270
   Ala Val Lys Gly Pro Tyr Phe Gly Asp Arg Arg Lys Ala Phe Leu Glu
           275                 280                 285
   Asp Leu Ala Val Val Thr Gly Gly Gln Val Val Asn Pro Asp Ala Gly
       290                 295                 300
   Met Val Leu Arg Glu Val Gly Leu Glu Val Leu Gly Ser Ala Arg Arg
   305                 310                 315                 320
   Val Val Val Ser Lys Asp Asp Thr Val Ile Val Asp Gly Gly Gly Thr
                   325                 330                 335
   Ala Glu Ala Val Ala Asn Arg Ala Lys His Leu Arg Ala Glu Ile Asp
               340                 345                 350
   Lys Ser Asp Ser Asp Trp Asp Arg Glu Lys Leu Gly Glu Arg Leu Ala
           355                 360                 365
   Lys Leu Ala Gly Gly Val Ala Val Ile Lys Val Gly Ala Ala Thr Glu
       370                 375                 380
   Thr Ala Leu Lys Glu Arg Lys Glu Ser Val Glu Asp Ala Val Ala Ala
   385                 390                 395                 400
   Ala Lys Ala Ala Val Glu Glu Gly Ile Val Pro Gly Gly Gly Ala Ser
                   405                 410                 415
   Leu Ile His Gln Ala Arg Lys Ala Leu Thr Glu Leu Arg Ala Ser Leu
               420                 425                 430
   Thr Gly Asp Glu Val Leu Gly Val Asp Val Phe Ser Glu Ala Leu Ala
           435                 440                 445
   Ala Pro Leu Phe Trp Ile Ala Ala Asn Ala Gly Leu Asp Gly Ser Val
       450                 455                 460
   Val Val Asn Lys Val Ser Glu Leu Pro Ala Gly His Gly Leu Asn Val
   465                 470                 475                 480
   Asn Thr Leu Ser Tyr Gly Asp Leu Ala Ala Asp Gly Val Ile Asp Pro
                   485                 490                 495
   Val Lys Val Thr Arg Ser Ala Val Leu Asn Ala Ser Ser Val Ala Arg
               500                 505                 510
   Met Val Leu Thr Thr Glu Thr Val Val Val Asp Lys Pro Ala Lys Ala
           515                 520                 525
   Glu Asp His Asp His His His Gly His Ala His
       530                 535

## Description

The present invention relates to the use of an approximately 60kDa polypeptide (or its encoding nucleic acid molecule) or functionally equivalent molecules or fragments thereof from *Mycobacterium tuberculosis* or related prokaryotes in the prevention and/or treatment of rheumatoid arthritis.

Autoimmunity reflects the loss of tolerance to "self' resulting in inappropriate destruction of normal cells or tissue. In many conditions, autoantibodies are found, but may reflect an effect rather than cause of a disease. In some diseases however autoantibodies are the first, major, or only detectable abnormality. One class of molecules which is implicated in this respect are the chaperonins which are highly immunogenic. Chaperonins belong to a group of proteins called molecular chaperones which bind non-native proteins and assist them, in an ATP-dependent catalytic process, to fold into the correct three-dimensional form required for a functional protein.

Chaperonins are believed to stimulate the immune system at many levels simultaneously, including monocytes, macrophages, fibroblast-like cells, perhaps other types of cells, and T cells. The immune defences in mammals may be divided into the "innate." and "adaptive" defences. Those which are already in place, such as phagocytes, natural killer cells and complement are considered innate. On challenge, adaptive immunity is activated in the form of B and T lymphocytes. Chaperonins are known to act directly on the innate defence mechanisms, particularly on phagocytes. They also stimulate a powerful adaptive immune response, namely the production of antibody and the stimulation of T lymphocytes which in some cases may be protective. Notably they induce cytokine secretion which is thought to be important for host defences. In some cases however it is believed that the presence of chaperonins may be damaging to the host.

Chaperonins' role in autoimmune disease is controversial. Although infection/immunity with chaperonin-containing organisms is universal, and healthy people have T cell responses to self-chaperonins, including the production of chaperonin-specific antibodies, classical autoimmune disease is quite uncommon. So the presence of immune reactions to chaperonins may be incidental and unimportant.

The theory of molecular mimicry however suggests the involvement of chaperonins in autoimmune disease and is based on the high level of amino acid sequence conservation between chaperonins of microbial and mammalian origin. The theory proposes that during infection with a wide range of microbes, chaperonin epitopes that are shared between microbes and mammals stimulate T lymphocytes. According to this theory a high level of chaperonin presentation of shared chaperonin epitopes breaks tolerance to self-chaperonins and autoimmune disease develops.

Chaperonins obtained from tumours have been found to result in necrotic effects on those tumours. It is suggested that this may be achieved through enhancing immunological recognition of tumour antigens although the mechanism of this is not known. It therefore appears that chaperonins induce protective adaptive immunity against bacterial infection and cancer.

Allergic reactions, such as asthma, concern proportionally inappropriate or misdirected immune responses. The prevalence of asthma for example is increasing and effective therapies for treating all cases have not yet been found. Current treatment often uses immunosuppressive glucocorticosteroids, beta agonists, cromoglycate, leukotriene modifiers etc. which have numerous side-effects.

In such allergic reactions, high IgE levels occur and T helper lymphocyte-2 (Th2) immune responses predominate over Th1 responses resulting in an inflammatory response. Th1 responses are thought to be mainly protective against microbial infection and are promoted by cytokines, particularly interleukin-12 (IL-12), IL-2 and interferon-γ. In contrast, Th2 responses, in the appropriate genetic background, are associated with harmful allergic tissue damage.

However, it has been suggested that in other conditions such as autoimmune disorders, e.g. adjuvant arthritis, overactive Th1 responses are causal of the disorder. Conversion of Th1 to Th2 or Th2 to Th1 responses may therefore have utility in treating the above described disorders.

Whilst it has been known that bacteria such as *L. monocytogenes, M. bovis and M. tuberculosis* can convert Th2 to Th1 responses, the molecules which is(are) responsible for this conversion have not been identified.

Suggestions in the art have however implicated a heat shock protein, hsp65, from *M. leprae* which is able to induce Th1 responses (Lowrie et al., 1999, Nature, 400, p269-271; Bonato et al., 1998, Infect Immun., 66, p169-175). The homologue, hsp65 from *M. tuberculosis,* has the ability to stimulate human monocytes to synthesize pro-inflammatory cytokines and activate monocytes and human vascular endothelial cells (Friedland et al., 1993, Clin. Exp. Immunol., 91, p5862; Peetermans et al., 1995, Infect. Immun., 63, p3454-3458; Verdegaal, et al., 1996, J. Immunol., 157, p369-376).

Surprisingly it has now been found that another protein which is not known to be a heat shock protein or a chaperonin is able to affect the immunity of an individual and can be used for treating or preventing non-cancerous conditions such as autoimmune disorders or conditions of immunoactivation, allergic conditions such as asthma and/or conditions typified by a Th2-type immune response and/or conditions associated with eosinophilia.

This protein of unknown function has been identified in *Mycobacterium tuberculosis* and sequenced (Kong et al., 1993, Proc. Natl. Acad. Sci., 90, p2608-2612). Comparable proteins are known to exist in various other bacteria, including *M. bovis* and *Legionella.* It has been named chaperonin 60.1 (cpn 60.1), but adoption of this nomenclature is simply based on its amino acid sequence identity to other chaperonins.

Chaperonin 60.2 (from the same source) exhibits 59.60% amino acid sequence identity and 65.6% nucleic acid sequence identity to cpn 60.1 using the alignment methods described hereinafter. Cpn 60.2 in common with cpn 60.1 does not have confirmed chaperonin properties. Chaperonins are believed to function by the formation of 2 ring heptamers (composed of approximately 60kDa monomers) which face one another and are capped by a ring heptamer composed of approximately 10kDa monomers (formed by cpn 10s). Assisted folding is achieved once the target protein has entered into the central core, whereafter it is released. Thus the formation of the heptamers appears to be essential to the presently understood functionality of chaperonins. However, unlike cpn 60s from other species, it has not been found possible to produce heptamers of *M. tuberculosis* cpn 60.1. Furthermore, unlike the GroE chaperonin folding machinery, neither the cpn 60.1 gene nor the cpn 60.2 gene is in the same operon as the chaperonin cpn 10 gene and thus transcription of the components which are necessary for the formation of a chaperonin complex is not under the same control mechanisms.

It has also been observed that the cpn 60.1 protein has a unique histidine-rich sequence at the C-terminus unlike cpn 60s from other species which usually have a sequence rich in glycine and methionine. The 60.2 protein is a known heat shock protein and has very high homology to related heat shock proteins in other species, e.g. 95% identity to the same protein from M. *leprae.* As mentioned above, cpn 60.2 is situated distant to cpn 60.1 on the genome of *M. tuberculosis* and is under distinct transcriptional control. As a consequence there is no evidence to suggest that cpn 60.1 is either a heat shock protein or a chaperonin. These facts strongly suggest different functional roles for the cpn 60 proteins in *M*. *tuberculosis.*
Kong et al. (1993) PNAS USA 90 pp2608-2612 identified and characterises a second chaparonin-60 homolog In *Mycobacterium tuberculosis* (Cpn 60.1).
Quayle et al. (1992) Euro. J. Immunol. 22 pp1315-1322 identifies T cell clones that interact with shared epitopes on human hsp 60 and Mycobacterium 65-kDa hsp.
Anderton et al. (1995) J. Exp. Med. 181 pp943-952 report that a peptide containing the 256-270 epitope from hsp65 was able to induce cross-reactive T cells that conferred protection against adjuvant arthritis.
Cobelens et al. (2000) Arthritis and Rheumatism 43 pp2694-2702 investigate the treatment of adjuvant induced arthritis by the oral administration of hsp65 during ongoing disease.
WO 00/27870 (Hadasit Medical Research) relates to peptides homologous to heat shock protein and DNA sequences encoding such peptides. The invention also relates to vaccines comprising such peptides.
WO 95/25744 (Rijksuniversiteit Utrecht) provides peptides for protection against or treatment of an inflammatory disease; the peptides are derived from microbial stress proteins.
Prakken et al. (1997) PNAS USA 94 pp3284-3289 investigated whether tolerance for an adjuvant arthritis associated T cell epitope from mycobacterial heat shock protein 60 can be obtained after intranasal administration of a peptide containing the hsp60 epitope.
Prakken et al. (1998) Biotherapy 10 pp205-211 investigated nasal administration of arthritis related T cell epitopes of hsp60 as a treatment for chronic arthritis.
Lewthwaite et al. (2001) Infection and Immunity 69 pp7349-7355 investigated the induction of cytokine synthesis by Cpn 60.1 and Cpn 60.2 from *M*. *tuberculosis.*
Baird et al. (1989) J. Gen. Microbial 135 pp931-939 characterises the 10 kDa antigen gene of *M. tuberculosis.*

The invention therefore provides molecules such as cpn 60.1 which have enhanced properties in treating or preventing rheumatoid arthritis.

Therapeutic and/or prophylactic applications may be achieved using nucleic acid molecules or peptides/proteins, as will be described in more detail hereinafter.

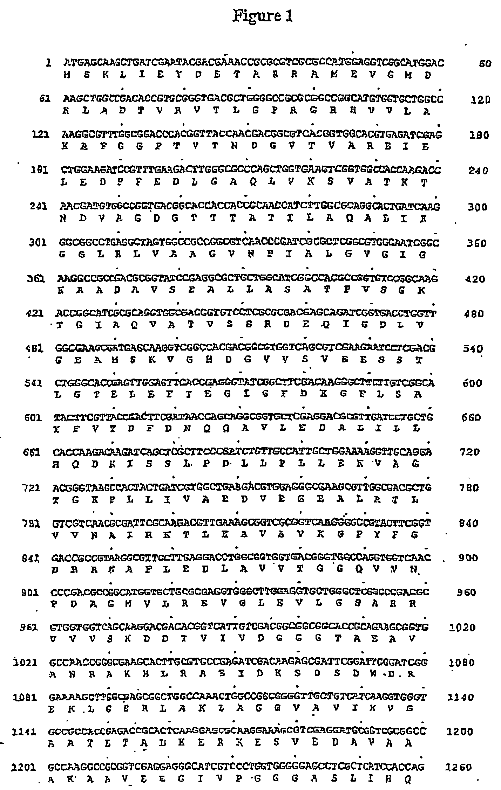

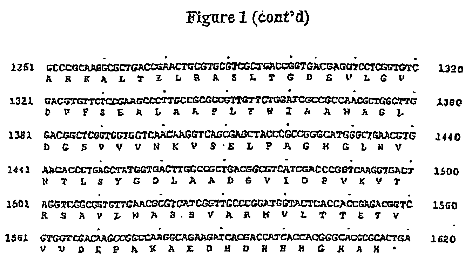

Thus, in a first aspect the present invention provides a pharmaceutical composition for use in the prevention and/or treatment of rheumatoid arthritis, wherein the pharmaceutical composition comprises a nucleic acid molecule comprising
(i) the nucleotide sequence or Figure 1, or
(ii) a sequence which has more than 70 e.g. 75%, preferably more than 80%, e.g. more than 90 or 95% identity to sequence (i) (according to the test described hereinafter) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or (iii) a fragment of sequence (i) or (ii) encoding a functionally equivalent protein fragment; and a pharmaceutically acceptable excipient, diluent or carrier.

As mentioned above, therapeutic and/or prophylactic effects may be achieved using nucleic acid molecules or peptide/protein molecules. Thus in a further aspect the present invention provides a pharmaceutical composition for use in the prevention and/or treatment of rheumatoid arthritis, wherein the pharmaceutical composition comprises a polypeptide comprising
(i) the amino acid sequence of Figure 1, or
(ii) a sequence which has more than 80%, e.g. more than 90 or 95% homology to sequence (i) (according to the test described hereinafter) which provides a functionally equivalent protein, or
(iii) a functionally equivalent fragment of sequence (i) or (ii); and a pharmaceutically acceptable excipient, diluent or carrier.

"Nucleic acid molecules" according to the invention may be single or double stranded DNA, cDNA or RNA, preferably DNA. Derivatives of nucleotide sequences capable of encoding functionally-equivalent polypeptides may be obtained by using conventional methods well known in the art.

Nucleic acid molecules for use in the invention may consist only of sequences derived from Figure 1 (or related functionally equivalent sequences), or may comprise additional sequences, such as structural or functional sequences, e.g. sequences which control transcription and/or expression (particularly in mammalian cells), or sequences which comprise the sequence for an additional protein moiety which may form a fusion protein which may have specific properties e.g. act as a secretory signal. Thus, for example, the sequence may be in the form of a vector containing the nucleic acid molecules described herein. Suitable vectors include plasmids and viruses.

"Polypeptides" as referred to herein includes both full-length protein and shorter length peptide sequences, e.g. protein fragments as described herein. Such polypeptides may be prepared by any convenient means, e.g. by isolation from the source prokaryote or by recombinant means by expression of the appropriate nucleic acid molecule in a host cell operatively linked to an expression control sequence, or a recombinant DNA cloning vehicle or vector containing such a recombinant DNA molecule or by chemical or biochemical synthesis (*ex vivo*).

"Sequence identity" as referred to herein in connection with nucleotide sequences refers to the value obtained when assessed using ClustalW (Thompson et al., 1994, Nucl. Acids Res., 22, p4673-4680) with the following parameters:
Pairwise alignment parameters - Method: accurate,
Matrix: JUB, Gap open penalty: 15.00, Gap extension penalty: 6.66; Multiple alignment parameters - Matrix: IUB, Gap open penalty: 15.00, % identity for delay: 30, Negative matrix: no, Gap extension penalty: 6.66, DNA transitions weighting: 0.5.
In connection with amino acid sequences, "sequence identity" refers to sequences which have the stated value when assessed using ClustalW (Thompson et al., 1994, supra) with the following parameters: Pairwise alignment parameters - Method: accurate,
Matrix: PAM, Gap open penalty: 10.00, Gap extension penalty: 0.10; Multiple alignment parameters - Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR. Sequence identity at a particular residue is intended to include identical residues which have simply been derivatized.

"Functionally equivalent" proteins or protein fragments refers to proteins or fragments related to, or derived from the amino acid sequence of Figure 1, where the amino acid sequence has been modified by single or multiple amino acid (e.g. at 1 to 50, e.g. 10 to 30, preferably 1 to 5 bases) substitution, addition and/or deletion but which nonetheless retains functional activity, e.g. suppresses ovalbumin-induced eosinophilia, for example reducing eosinophil numbers to the extent of more than 10 %, e.g. more than 25%, particularly preferably more than 50% and/or an increase in the production of specific cytokines such as interleukin-1*β* (IL-1*β*), IL-2, IL-6, IL-8, IL-10, IL-12, IL-12 receptor, tumour necrosis factor a (TNFα), interferon-γ and granulocyte-macrophage-colony stimulating factor (GM-CSF) e.g. a more than 10 fold, preferably more than 100 fold increase over normal levels and/or stimulation of Th1 responses. Cytokine stimulation can be measured by a variety of methods. For example, Buffy coat blood is diluted 3-fold with PBS-2% fetal calf serum (FCS). 30 ml is layered on 15 ml of Lymphoprep (Histopaque 1077) and centrigued at room temperature for 30 min at 700 g (1800 rpm, Eppendorf centrifuge). The layer of mononuclear cells is aspirated carefully and the cells washed twice in PBS. The cells are finally resuspended at 2 x 10⁶ cells/ml. PBMC's are subsequently seeded at 2 x 10⁶ cells/well in RPMI medium with 2% FCS, glutamine and Pen/strep and incubated for 1 h to let monocytes adhere to the plate surface. The plates are washed one with PBS.
PBMC's depleted for T cells are obtained in the same way with the sole difference of an initial incubation with the RosetteSep reagent (Stemcell) for 20 min at room temperature.
Cytokine assays are within the knowledge of skilled persons. For example, IL6 and IL8 production can be measured after diluting the cell supernatant 1/10 and 1/100 respectively. The paired antibodies and standards may be obtained from the National Institute for Biological Standards and Control and used as recommended.
Sample preparation can be as follows: Cpn60.1 and Cpn60.2 can be obtained from Lionex (Germany). Both proteins are diluted to a concentration to 200 µg/ml in PBS prior to boiling or autoclaving. The boiled samples are obtained by incubation at 100°C for 20 min and subsequently directly placed on ice. The autoclaved sample is obtained by autoclaving at 120°C for 20 min twice. SDS-PAGE can be performed on 4-20% gradient gels (Invitrogen, Netherlands). The prestained protein marker is the Benchmark Prestained Protein Ladder from Gibco/BRL. FACS analysis can be performed on a FacsCan apparatus (Becton Dickinson) and the data analysed using the WinMDI program version 2.8.

Within the meaning of "addition" variants are included amino and/or carboxyl terminal fusion proteins or polypeptides, comprising an additional protein or polypeptide fused to the polypeptide sequence.

Particularly preferred are naturally occurring equivalents such as biological variations, e.g. allelic, geographical or allotypic variants and derivatives prepared using known techniques. For example, functionally-equivalent proteins or fragments may be prepared either by chemical peptide synthesis or in recombinant form using the known techniques of site-directed mutagenesis, random mutagenesis, or enzymatic cleavage and/or ligation of nucleic acids.

The disclosure is particularly directed to homologues and related molecules from different prokaryotes, e.g. from bacterial genera, species or strains, particularly from the genus *Mycobacterium, e.g.* homologues from the *Mycobacterium tuberculosis* complex which includes *M*. *tuberculosis, M. bovis* and *M. africanum.* Such sequences may themselves be modified, particularly derivatized providing they still retain functionality.

Derivatives of the proteins may be prepared by post-synthesis/isolation modification or by modification during synthesis, e.g. using modified residues or expression of modified nucleic acid molecules, where appropriate.

Functionally-equivalent fragments according to the invention may be made by truncation, e.g. by removal of a peptide from the N and/or C-terminal ends or by selection of an appropriate active domain region, e.g. an epitopic region which retains its functionality. Such fragments may be derived from the sequence of Figure 1 or may be derived from a functionally equivalent protein to that disclosed in Figure 1.

It will be appreciated that where functional fragments are selected they may not exhibit all functions attributed to the source molecules. Thus functionally equivalent proteins or fragments refers to retention of relevant functional properties such that the fragment retains utility according to the invention, e.g. reduces eosinophilia, increases the production of specific cytokine and/or stimulates the Th1 immune response, as mentioned above.

Preferably the fragments are between 6 and 400 residues in length, e.g. 6 to 100 or 15 to 100 residues, preferably 6 to 30, 10 to 25, 15 to 50 or 15 to 30 residues. Particularly preferred fragments are those derived from or consisting of residues:

| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD (12) |
| 327-342 | DTVIVDGGGTAEAVAN, (16) |
| 343-363 | RAKHLRAEIDK, (11) |
| 379-391 | VGAATETALKERK (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVKVSE, (8) |
| 480-494 | VNTLSYGDLAADGVI, (15) |
| 501-526 | RSAVLNASSVARMVLTTETVVVDKPA, (15) |
| 526-539 | KAEDHDHHHGHAH. (14) |

Functionally equivalent nucleic acid sequences/fragments compared to the sequence recited in Figure 1 are also used in compositions of the invention. These sequences are defined with reference to the functionally equivalent protein/peptides (as defined above) which they encode.

"Hybridisation" as used herein refers to those sequences which bind under non-stringent conditions (6 x SSC/50% formamide at room temperature) and washed under conditions of high stringency e.g. 2 x *SSC,* 65°C (where *SSC* = 0.15M NaCl, 0.015M sodium citrate, pH 7.2).

"Pharmaceutically acceptable" as referred to herein refers to ingredients that are compatible with other ingredients of the compositions as well as physiologically acceptable to the recipient.

Pharmaceutical compositions according to the invention may be formulated in conventional manner using readily available ingredients. Thus, the active ingredient (ie. the nucleic acid molecule or protein/peptide), may be incorporated, optionally together with other active substances, with one or more conventional carriers, diluents and/or excipients, to produce conventional galenic preparations such as tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, sterile packaged powders, and the like.

As mentioned above, compositions may additionally comprise molecules which assist or augment the action of the nucleic acid molecules or polypeptides described hereinbefore, e.g. thalidomide (and analogues thereof), low dose cyclophosphamide, LPS, cytokines, chemokines, CpG oligodeoxynucleotides and other immunomodulators and/or antiinflammatory agents such as cytokine antagonists or glucocorticosteroids.

Thus for example, the compositions may be used together with active ingredients for specific immunotherapies. Appropriate immunotherapy treatment/vaccine preparations which may include nucleic acid molecules/polypeptides as described herein include subunit vaccines or treatments based on cell specific antigens or associated antigens or antibody, anti-idiotype antibody or whole cell preparations for vaccination or therapy. When used in therapy or vaccination the nucleic acid molecules or polypeptides described herein may provide (or encode) an antigen resulting in a specific immune response directed to that antigen and/or may result in a general and nonspecific immune response. In the latter case in which compositions containing other active ingredients are used, the nucleic acid molecules/polypeptides described herein act as adjuvants and may be used for this purpose.

Preventative or therapeutic preparations may be formulated to include one or more suitable adjuvants, e.g. Incomplete Freund's Adjuvant, BCG, Montanide, aluminium hydroxide, saponin, quil A, or more purified forms thereof, muramyl dipeptide, mineral or vegetable oils, Novasome or non-ionic block co-polymers or DEAE dextran, in the presence of one or more pharmaceutically acceptable carriers or diluents. Suitable carriers include liquid media such as saline solution.

Examples of suitable carriers, excipients, and diluents are lactose, dextrose, sucrose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/glycol, water/polyethylene glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The compositions of the invention may be formulated so as to provide quick, sustained or delayed release of the active ingredient after administration to the patient by employing procedures well known in the art.

Compositions may be in an appropriate dosage form, for example as an emulsion or in liposomes, niosomes, microspheres, nanoparticles or the like.

If required, the compositions may also contain targeting moieties attached to the active ingredient, e.g. a ligand which binds specifically and selectively to an endogenous receptor to allow targeting to a particular cell type or location, such as targeting to lymphocytes, monocytes, macrophages, endothelial cells, epithelial cells, blood cells, erythrocytes, platelets, eosinophils, neutrophils, natural killer cells, dendritic cells, brain cells, heart cells, lung cells, islet cells, kidney cells, hormonal gland cells, skin, bone, joints, bone marrow, gastric mucosa, lymph nodes, Peyers patches, the omentum and other immunological tissues.

As defined herein "treatment" refers to reducing, alleviating or eliminating one or more symptoms of the condition which is being treated, relative to the symptoms prior to treatment.

"Prevention" of a condition refers to delaying or preventing the onset of a condition or reducing its severity, as assessed by the appearance or extent of one or more symptoms of said condition.

In particular, non-cancerous conditions which may be treated include rheumatoid arthritis.

Patients which may be treated include, but are not limited to mammals, particularly primates, domestic animals and livestock. Thus preferred animals for treatment include mice, rats, guinea pigs, cats, dogs, pigs, goats, sheep, horses and particularly preferably, humans.

As mentioned previously, either nucleic acid molecules or polypeptides may be used in the methods of the invention. In instances in which nucleic acid molecules are employed, these are conveniently applied in a form to allow their expression within the patient, thus providing a form of gene therapy. Thus the pharmaceutical compositions described herein containing a nucleic acid molecule may be used in methods of gene therapy.

Thus for example the nucleic acid molecules may be provided in a liposome, micelle or other convenient carrying vehicle which may comprise targeting moieties to allow its targeting to cells of interest.

Alternatively the molecules may be packaged in other, "vehicles" such as viruses, plasmids or cells (particularly transfected species-matched cells) which are all well known in the art for this purpose which allow expression of the resident molecule.

Appropriate techniques for transfection are well known and include electroporarion, microinjection, lipofection, adsorption, viral transfection and protoplast fusion.

Administration of compositions of the invention may take place by any of the conventional routes, e.g. by inhalation, nasally, orally, rectally or parenterally, such as by intramuscular, subcutaneous, intraperitoneal or intravenous injection. Treatment or prophylaxis by topical application of a composition, e.g. an ointment, to the skin is also possible. Optionally administration may be performed at intervals, e.g. 2 or more applications, e.g. 2-4 applications at hourly, daily, weekly or monthly intervals, e.g. several times a day, or every 3-5 days, or at fortnightly, monthly or quarterly intervals.

It has been observed in work conducted on the related molecule cpn 60.2 that the route of administration may affect the immune response which is generated. For example when Mtcpn 60.2 is administered intranasally, a Th2 to Th1 shift is stimulated although the reverse effect is observed when administered intraperitoneally. Thus, the route of administration should take into account the disorder to be treated/prevented and thus for example in treating autoimmune disorders, intraperitoneal administration may be appropriate whereas treatment or prevention of particularly allergic disorders may be for example by intranasal administration.

In prophylactic methods of the invention, administration (conveniently orally or by inhalation or subcutaneous or intramuscular injection) is preferably performed at more lengthy intervals, e.g. intervals of 2-12 weeks. For therapeutic purposes, administration (conveniently orally or by inhalation or intravenous injection) is performed 1-4 times in a single day or over 2 days.

The active ingredient in composition of the invention may comprise from about 0.01% to about 99% by weight of the formulation, preferably from about 0.1 to about 50%, for example 10%. The compositions are preferably formulated in a unit dosage form, e.g. with each dosage containing from about 0.01mg to about 1g of the active ingredient, e.g. 0.05mg to 0.5g, for a human, e.g. 1-100mg.

The precise dosage of the active compound to be administered and the length of the course of treatment will, of course, depend on a number of factors including for example, the age and weight of the patient, the specific condition requiring treatment and its severity, and the route of administration. Generally however, an effective dose may lie in the range of from about 0.1µg/kg to about 14mg/kg, preferably 0.1 to 1mg/kg, e.g. from about 1mg to 1g of polypeptide per day, depending on the animal to be treated and the dosage form, taken as a single dose. Thus for example, an appropriate daily dose for an adult may be from 7µg to 1g, e.g. 10mg to 1g per day, e.g. 25 to 500mg of the polypeptide per day.

Similar or lower dosages may be used when using nucleic acid molecules described herein, e.g. from about 0.2ng/kg to about 2.5mg/kg (e.g. from about 0.2ng/kg to about 2ng/kg or about 1.5ng/kg to about 2.5mg/kg) such as about 14ng to about 175mg for an adult. However, where the nucleic acid molecules are packaged in cells or vectors proportionally higher or lower amounts may be required depending on the extent of non-cpn encoding DNA and sequences which influence the level of expression, e.g. 5 or 10-fold larger amounts, e.g. nucleic acid molecules described herein packaged in a vector may be used at about 1.0ng/kg to about 12.5mg/kg.

As mentioned above, the family of polypeptides defined herein and the nucleic acid molecules encoding them stimulate the production of a set of cytokines. This therefore allow the use of these compounds for the express purpose of stimulating production of these cytokines whether or not this occurs in a therapeutic/prophylactic situation.

Preferably the cytokines which are increased, e.g. more than 10 or 100 fold relative to normal levels, are selected from the group consisting of IL-1β, IL-2, IL-6, IL-8, IL-10, IL-12, TNFα, interferon-γ and GM-CSF.

### Definitions

*"ADJUVANT".* This term is intended to cover any substance which, when incorporated into or administered simultaneously with antigen, potentiates the immune response.

*"MT60.1",* "Mtcpn60.1", "cpn 60.1", "60.1" are used interchangeably throughout the specification to refer to the amino acid sequence shown in Figure 1.

### Description of Figures

Figure 1 shows the nucleotide and amino acid sequence of cpn 60.1 from *M. tuberculosis;*
Figure 2 shows the *in vitro* effects of *M. tuberculosis* cpn 60.1 and 60.2 on cytokine production; A) IL-1β, B) IL-6, C) IL-8, D) IL-10, E) IL-12, F) TNFα and G) GM-CSF, wherein the filled circles represent cpn 60.1 and the open circles represent cpn 60.2;
Figure 3 shows ELISA results which indicate a significant antibody generation in chaperonin-treated rats (ie 60.1 and 60.2 treated animals), but not in untreated, or PBS treated animals. Key: Naive (no immunisation); Antigen used in ELISA is cpn 60.2 ("65"); cpn 60.1 ("cpn") or cpn 10 ("10"). For example, on the y axis "Naïve/65" indicates that the animal was not immunised and the antigen used in the ELISA was 60.2. Similarly, "cpn/cpn" indicates that the animal was immunised with 60.1 and that the antigen used in the ELISA was 60.1.
Figure 4 shows the effect, in terms of clinical score for arthritis, of treatment with and without cpn60.1, 60.2 or 10.
Figure 5 shows the effect, in terms of x-ray score for arthritis, of treatment with and without cpn60.1, 60.2, or 10. Key: "Ost" = osteoporosis; "Peri" = perioditis; and "Ero" = erosion.

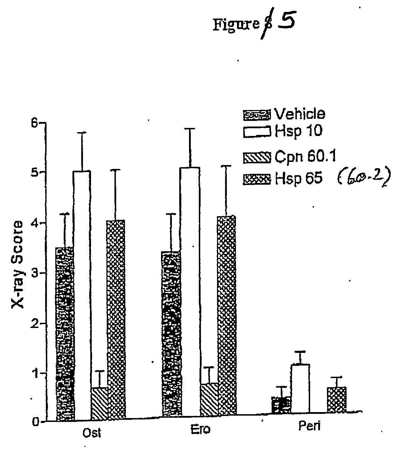

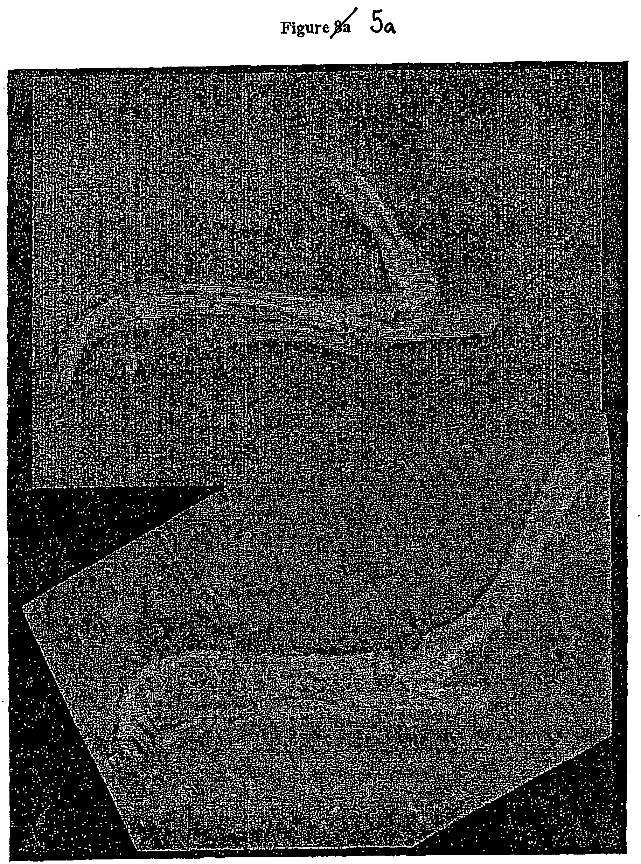

Figure 5(a) shows the effect of cpn60.1 treatment of adjuvant-induced rheumatoid arthritis in the Wistar rat. Arthritis was induced by a single intra-dermal tail injection of heat-killed *Mycobacterium tuberculosis* in oil (adjuvant). Treatment was by injection of CPN 60.1 (50µg in phosphate buffered saline) on days 4, 5 and 6 after induction. Disease progression, assessed on paw inflammation, was monitored daily. X-ray scans were taken when arthritis was maximal (>9 days following treatment). Upper scan: rat rear paw treated with CPN 60.1 post induction with adjuvant, showing bone density and joint physiology indistinguishable from normal rats. Lower scan: rat rear paw showing rheumatoid lesions typical in adjuvant-induced arthritis: bone erosion, osteoporosis, joint changes and occlusion. These are annotated in
Figure 5b.

### EXAMPLE 1: Mycobacterium tuberculosis cpn 60.1 is a powerful inducer of cytokines

In this experiment the cytokine inducing activity of purified *M. tuberculosis* cpn 60.1 recombinant proteins were examined by ELISA.

### Methods

*Expression and purification of chaperonin 60 proteins M. tuberculosis* cpn 10, 60.1 and 60.2 were prepared by Prof M. Singh (WHO Collaborating Centre, Germany) using conventional chromatography as described below.

### Purification of recombinant cpn 60.2:

The protein was obtained from heat-induced (42°C) recombinant *E.coli* K12 cells that carry a plasmid encoding *M. tuberculosis* cpn 60.2. Cells were lysed by sonication. The supernatant was chromatographed on an anion exchange chromatography column. After dialysis the fractions containing cpn 60.2 were further purified on a second different anion exchange chromatography column. Finally the protein solution was dialysed against 10mM ammonium bicarbonate before aliquotting and lyophilization.

Great care was taken to check each batch of protein for LPS contamination using the Limulus assay (Tabona et al., 1998, J. Immunol., 16], p1414-1421). If LPS contamination was detected it was removed on a polymyxin B affinity column and levels of LPS re-assayed. Recombinant, LPS-low, chaperonins were further purified on a Reactive Red column to remove contaminating proteins and peptides (Tabona et al., 1998, supra).

The *in vitro* effects of cpn 60.1 and cpn 60.2 on the production of IL-1β, IL-6, IL-8, IL-10, IL-12, TNFα and GM-CSF in human PBMCs was determined using 2-site ELISA as described by Tabona et al., 1998, supra.

### Results

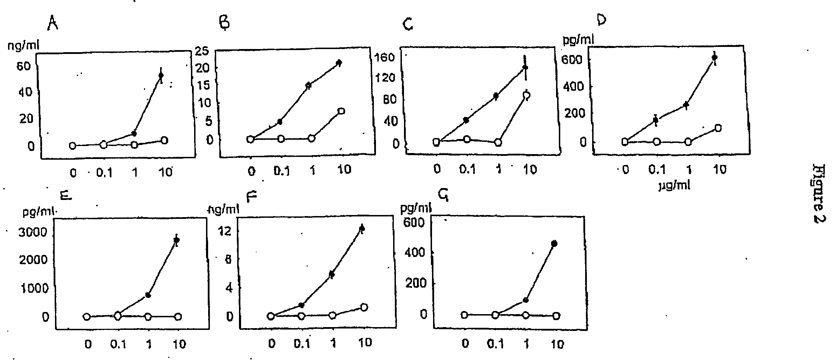

The results are shown in Figure 2. Surprisingly, the cpn 60.1 protein proved to be a much more potent cytokine inducer than the well-studied cpn 60.2 or hsp65. In addition to being two log orders more potent than cpn 60.2, the cpn 60.1 protein stimulates a significantly greater maximal response in human monocytes than does cpn 60.2 or LPS. Of interest, both chaperonins stimulate IL-12 production but fail to promote the formation of the anti-mycobacterial cytokine IFN-γ. In this context we have examined a number of cpn 60-derived peptides. The putative cpn 60.1 T cell epitope peptide, 195-219, proved to be a potent inducer of cytokine synthesis, including IFN-γ (data not shown). The same peptides in *M*. *tuberculosis* cpn 60.2 and in the *E*. *coli* cpn 60 protein, groEL, were without cytokine-inducing activity (Lewthwaite, Henderson and Coates, unpublished data). These findings confirm the action of *M. tuberculosis* cpn 60.1 on monocytes/macrophages and thus their use in the prevention or treatment of certain conditions.

### Example. 2: Autoimmune Disease: 60.1 suppresses arthritis in the rat.

This example shows for the first time that in a rat model of arthritis *M. tuberculosis* cpn 60.1 protein inhibited osteoporosis bone erosions and periostitis in immunized rats. These data support show that Mtcpn 60.1 modulates arthiritis in the rat and therefore, has important implications for arthritis disease treatment and prevention.

### Methods

While adjuvant/*M*. *tuberculosis* is not a stimulus often encountered by arthritis subjects, the Th1 responses observed in rat models of inflammation are analogous to those observed in rheumatoid arthritis.

The Th1 cytokine profile generated by both allergens are similar. The advantage of using this adjuvant is that it is easily available and the specific activity of this stimulus does not change between batches and therefore, we can control for adjuvant dose between batches.

### General Protocol for Induction of Adjuvant Arthritis

Heat killed human strains C, DT and PN of *Mycobacterium tuberculosis* (Mtb) are finely ground in a pestle and mortar and suspended in light paraffin oil to a final concentration of 10mg/ml.

The rats are inoculated at the base of the tail with a total of 100 µl of the Mtb suspension (Andrews *et al,* 1987). Animals are observed daily for 3-4 weeks, assessing the body weights and clinical scores. At the end of the experiment, animals are killed by asphyxiation in CO₂ and blood and tissue samples collected.

### Clinical scores

The day or arthritis induction is designated as day 0 and arthritis evaluated using the following standard scoring system (adapted from the work of Currey and Ziff, 1968).

### 0. No inflammation.

1. Slight redness and swelling of the foot.
2. Swelling of the foot such that the tendons are no longer visible.
3. Swelling extending to the ankle joint.
4. Gross inflamation and deformity of the ankle joint

Scores are summed for each animal giving a potential maximum of 16.

Additionally, the tail can be scored 0 to 1 according to the absence or presence of cutaneous nodules and the ears scored 0 to 1 according to the absence or presence of redness. Tail and ears were not scored in the chaperonin experiment.

### References

Andrews FJ, Morris CJ, Kondratowicz B, Bake DR: Effect of iron chelation on inflammatory joint disease. Ann Rheum Dis 1987; 46: 327-33.
Currey HLF, Ziff M. Suppression of adjuvant disease in the rat by heterologous antilymphocyte globulin. J Exp Med 1968; 127: 185-203.

### Experimental details

Species: Wistar rat 160-200g at start of experiment
Strain: Bath - bom and weaned
Sex: Female
No in cage: 6

### Heat shock protein (chaperonin) preps:

Cpn 10 (hsp 10): supplied at 1mg/ml in 20 mM potassium phosphate, 1mM DTT, 1mM EDTA (1ml)
Cpn 60.1 (hsp60.1): supplied at 2.25 mg/ml in 10mM ammonium bicarbonate (0.3ml)
Cpn 60.2 (hsp65): supplied at 3.33 mg/ml in 10 mM ammonium bicarbonate (0.45ml)

### Phosphate Buffered Saline (PBS)

### Sterile Dulbecco's PBS w/o Ca and Mg used (Gibco)

Added 720µl PBS to Cpn 60.1 prep, 570µl to Cpn 60.2 prep and 120µl to hsp 10 prep (under volume) to make up to 1mg/ml; cpn preps aliquoted (320µl) into eppendorfs and kept in fridge until injected. Remainder refrozen at - 70°C for ELISAs.

Five groups of 6 rats were inoculated with 100µl pulverised heat killed Mtb (10mg/ml) in light paraffin oil (adjuvant) in the tail (Day 0). One group of 6 rats received no treatment (naïve). All were weighed. On days 4, 5 and 6, the 5 groups receiving adjuvant were treated as follows:
Group 1: no treatment (AA Alone)
Group 2: each rat was injected with 50 µl PBS in the tail (AA + vehicle)
Group 3: each rat was injected with 50 µl hsp 10 in the tail (AA + hsp 10)
Group 4: each rat was injected with 50 µl cpn60.1 in the tail (AA + cpn60.1)
Group 5: each rat was injected with 50 µl hsp65 (cpn60.2) in the tail (AA + hsp65)
Group 6: Naive

All animals were scored daily and weighed on days 0, 4, 6, 8,11, 13, 15, 18 and 21.

### Results

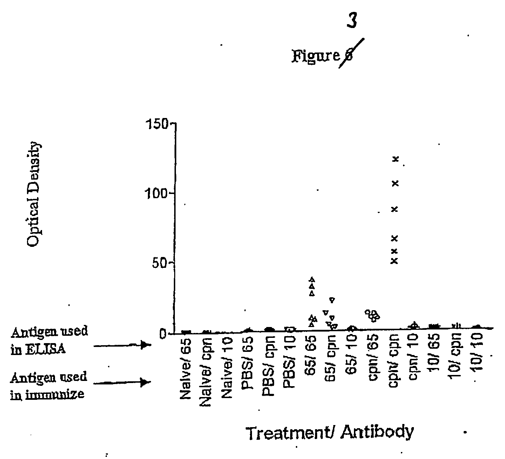

Figure 3 shows a significant antibody generation in chaperonin-treated rats, but not in untreated or PBS-treated animals.

Figure 3 also shows that immunization of adjuvant-treated rats with cpn60.1 provokes the largest amount of antibody, which suggests induction of a Th2-response.

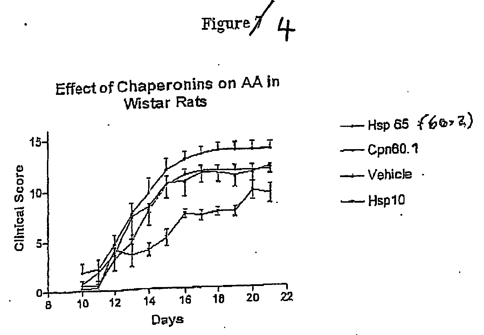

Figure 4 shows the results obtained with and without treatment with cpn 60.1, 60.2 or 10. Treatment with vehicle alone gave a clinical score of 12 after 21 days. This was increased by 60.2 to 14 and was reduced by cpn 10 to 9. Treatment with 60.1 was indistinguishable from vehicle alone. This provides evidence that the inflammatory response is increased by 60.2, decreased by 10, whilst 60.1 has no effect.

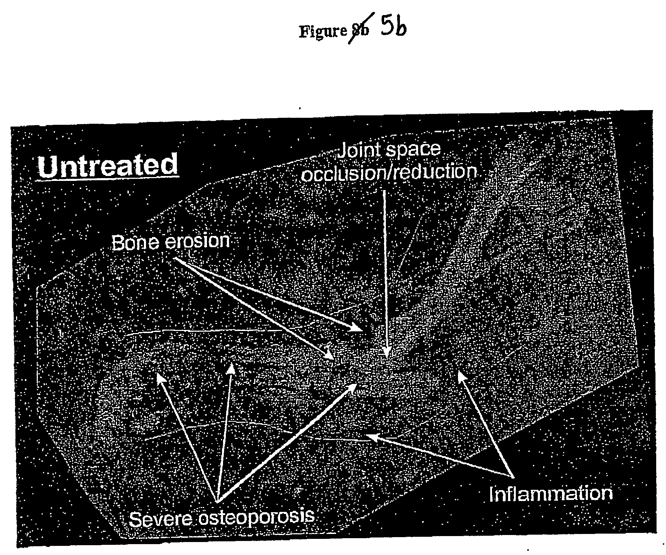

The applicants have also examined the joints by x-ray (Fig 5, 5(a) and 5(b)). 10 increased the x-ray scores for osteoporosis, bone erosions and periostitis; 60.2 was no different to vehicle alone.

These data demonstrate for the first time that Mtcpn 60.1 can suppress osteoporosis, bone erosions and periostitis in a rat model of arthritis. This shows that this protein has the potential to modulate arthritis in the rat, which has important implications for the treatment and prevention of autoimmune and allergic diseases.

Autoimmune disease, such as arthritis, is thought to operate by a mechanism (over-reactivity of Th-1 cells) different from allergic conditions (over-reactivity of Th-2 cells). It is particularly surprising therefore that 60.1 suppresses *both* asthma (Th2) and arthritis (Th1).

## Claims

1. A pharmaceutical composition for use in the prevention and/or treatment of rheumatoid arthritis wherein the pharmaceutical composition comprises a nucleic acid molecule comprising
(i) the nucleotide sequence of Figure 1, or
(ii) a sequence which has more than 70 e.g. 75%, preferably more than 80%, e.g. more than 90 or 95% identity to sequence (i) or a sequence which hybridizes to sequence (i) under conditions of 2 x SSC, 65 °C (wherein SCC = 0.15M NaCl, 0.015M sodium citrate, pH 7.2) which encodes a functionally equivalent protein to the sequence encoded by the nucleotide sequence of Figure 1, or
(iii) a fragment of sequence (i) or (ii) encoding a functionally equivalent protein fragment; and a pharmaceutically acceptable excipient, diluent or carrier.

2. A pharmaceutical composition for use in the prevention and/or treatment of rheumatoid arthritis wherein the pharmaceutical composition comprises a polypeptide comprising
(i) the amino acid sequence of Figure 1, or
(ii) a sequence which has more than 80%, e.g. more than 90 or 95% homology to sequence (i) which provides a functionally equivalent protein, or
(iii) a functionally equivalent fragment of sequence (i) or (ii); and a pharmaceutically acceptable excipient, diluent or carrier.

3. The pharmaceutical composition for use according to Claim 2 wherein the fragments are between 6 and 400 residues in length.

4. The pharmaceutical composition for use according to Claim 3 wherein the fragment lengths are between 6 to 100 or 15 to 100 residues.

5. The pharmaceutical composition for use according to Claim 4, wherein the fragment lengths are between 6 to 30, 10 to 25, 15 to 50 or 15 to 30 residues.

6. The pharmaceutical composition for use according to Claim 2 wherein the fragments are derived from or consisting of at least one of the following sequences
| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) (25) |
| 195-219 | KGFLSAYFVTDFDNQQAVLEDALIL, |
| 206-219 | FDNQQAVLEDAALIL, (14). |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD, (12) |
| 327-342 | DTVIVDGGGTAEAVAN, (16) |
| 343-353 | RAKHLRAEIDK, (11) |
| 379-391 | VGAATETALKERK, (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 480-494 | VNTLSYGDLAADGVI, (15) |
| 501-526 | RSAVLNASSVARMVLTTETVVVDKPA, (15) |
| 526-539 | KAEDHDHHHGHAH, (14) |

7. The pharmaceutical composition for use according to any one of Claims 1 to 6 wherein a therapeutically or prophylactically effective dose, or plurality of doses, of the pharmaceutical composition are administered.

## Patentansprüche

1. Pharmazeutische Zusammensetzung zur Verwendung bei der Prävention und/oder Behandlung von rheumatoider Arthritis, wobei die pharmazeutische Zusammensetzung ein Nukleinsäuremolekül umfasst, welches Folgendes umfasst:
(i) die Nukleotidsequenz von Fig. 1 oder
(ii) eine Sequenz mit mehr als 70%, z.B. 75%, vorzugsweise mehr als 80%, z.B. mehr als 90% oder 95% Identität zur Sequenz (i) oder eine Sequenz, welche unter Bedingungen von 2 x SSC, 65°C (wobei SCC = 0,15 M NaCl, 0,015 M Natriumcitrat, pH-Wert 7,2) mit Sequenz (i) hybridisiert, welche für ein der durch die Nukleotidsequenz von Fig. 1 codierten Sequenz funktionell äquivalentes Protein codiert, oder
(iii) ein Fragment von Sequenz (i) oder (ii), welches für ein funktionell äquivalentes Proteinfragment codiert, und einen pharmazeutisch unbedenklichen Exzipienten, ein pharmazeutisch unbedenkliches Verdünnungsmittel oder einen pharmazeutisch unbedenklichen Träger.

2. Pharmazeutische Zusammensetzung zur Verwendung bei der Prävention und/oder Behandlung von rheumatoider Arthritis, wobei die pharmazeutische Zusammensetzung ein Polypeptid umfasst, welches Folgendes umfasst:
(i) die Aminosäuresequenz von Fig. 1 oder
(ii) eine Sequenz mit mehr als 80%, z.B. mehr als 90% oder 95%, Homologie zur Sequenz (i), welche ein funktionell äquivalentes Protein bereitstellt, oder
(iii) ein funktionell äquivalentes Fragment von Sequenz (i) oder (ii), und einen pharmazeutisch unbedenklichen Exzipienten, ein pharmazeutisch unbedenkliches Verdünnungsmittel oder einen pharmazeutisch unbedenklichen Träger.

3. Pharmazeutische Zusammensetzung zur Verwendung nach Anspruch 2, wobei die Fragmente eine Länge von 6 bis 400 Resten aufweisen.

4. Pharmazeutische Zusammensetzung zur Verwendung nach Anspruch 3, wobei die Fragmente Längen von 6 bis 100 oder 15 bis 100 Resten aufweisen.

5. Pharmazeutische Zusammensetzung zur Verwendung nach Anspruch 4, wobei die Fragmente Längen von 6 bis 30, 10 bis 25, 15 bis 50 oder 15 bis 30 Resten aufweisen.

6. Pharmazeutische Zusammensetzung zur Verwendung nach Anspruch 2, wobei die Fragmente von mindestens einer der folgenden Sequenzen abgeleitet sind bzw. daraus bestehen:
| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ,(12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD, (12) |
| 327-342 | DTVTVDGGGTAEAVAN, (16) |
| 343-353 | RAKHLRAEID, (11) |
| 379-391 | VGAATETALKERK, (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 490-494 | VNTLSYGDLAADGVI, (15) |
| 501-526 | RSAVLNASSVARMVLTTETVVVDKPA, (15) |
| 526-539 | KAEDHDHHHGHAH, (14) |

7. Pharmazeutische Zusammensetzung zur Verwendung nach einem der Ansprüche 1 bis 6, wobei eine therapeutisch oder prophylaktisch wirksame Dosis oder mehrere Dosen der pharmazeutischen Zusammensetzung verabreicht werden.

## Revendications

1. Composition pharmaceutique pour utilisation dans la prévention et/ou le traitement de la polyarthrite rhumatoïde, **caractérisée en ce que** la composition pharmaceutique comprend une molécule d'acide nucléique comprenant
(i) la séquence de nucléotides de la figure 1, ou
(ii) une séquence qui a plus de 70, par exemple 75 %, de préférence plus de 80 %, par exemple plus de 90 ou 95 % d'identité avec la séquence (i) ou une séquence qui s'hybride avec la séquence (i) dans des conditions de 2 x SSC, 65 °C (où SCC = NaCl 0,15 M, citrate de sodium 0,015 M, pH 7,2) qui code pour une protéine fonctionnellement équivalente à la séquence codée par la séquence de nucléotides de la figure 1, ou
(iii) un fragment de séquence (i) ou (ii) codant pour un fragment de protéine fonctionnellement équivalent ; et un excipient, diluant ou véhicule pharmaceutiquement acceptable.

2. Composition pharmaceutique pour utilisation dans la prévention et/ou le traitement de la polyarthrite rhumatoïde **caractérisée en ce que** la composition pharmaceutique comprend un polypeptide comprenant
(i) la séquence d'acides aminés de la figure 1, ou
(ii) une séquence qui a plus de 80 %, par exemple plus de 90 ou 95 % d'homologie avec la séquence (i) qui produit une protéine fonctionnellement équivalente, ou
(iii) un fragment fonctionnellement équivalent de la séquence (i) ou (ii) ; et un excipient, diluant ou véhicule pharmaceutiquement acceptable.

3. Composition pharmaceutique pour utilisation selon la revendication 2 dans laquelle les fragments sont compris entre 6 et 400 résidus de longueur.

4. Composition pharmaceutique pour utilisation selon la revendication 3 dans laquelle les longueurs de fragment sont comprises entre 6 et 100 ou 15 et 100 résidus.

5. Composition pharmaceutique pour utilisation selon la revendication 4, dans laquelle les longueurs de fragment sont comprises entre 6 et 30, 10 et 25, 15 et 50 ou 15 et 30 résidus.

6. Composition pharmaceutique pour utilisation selon la revendication 2 dans laquelle les fragments sont dérivés de ou constitués d'au moins l'une des séquences suivantes :
| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD, (12) |
| 327-342 | DTVIVDGGGTAEAVAN, (16) |
| 343-353 | RAKHLRAEIDK, (11) |
| 379-391 | VGAATETALKERK, (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 480-494 | VNTLSYGDLAADGVI, (15) |
| 501-526 | RSAVLNASSVARMVLTTETVVVDKPA, (15) |
| 526-539 | KAEDHDHHHGHAH, (14) |

7. Composition pharmaceutique pour utilisation selon l'une quelconque des revendications 1 à 6 **caractérisée en ce qu'**une dose, ou une pluralité de doses, thérapeutiquement ou prophylactiquement efficaces de la composition pharmaceutique sont administrées.
